(19) Europäisches Patentamt
European Patent Office
Office européen des brevets

(11) Veröffentlichungsnummer: **0 051 557**
**B1**

# (12) EUROPÄISCHE PATENTSCHRIFT

(45) Veröffentlichungstag der Patentschrift: **14.03.84**

(51) Int. Cl.³: **C 07 D 307/935,** A 61 K 31/34 // C07F7/18, C07F9/42

(21) Anmeldenummer: **81730113.8**

(22) Anmeldetag: **29.10.81**

## (54) 5-Cyan-prostacyclinderivate, Verfahren zu ihrer Herstellung und ihrer Verwendung als Arzneimittel.

(30) Priorität: **31.10.80 DE 3041602**

(43) Veröffentlichungstag der Anmeldung: **12.05.82 Patentblatt 82/19**

(45) Bekanntmachung des Hinweises auf die Patenterteilung: **14.03.84 Patentblatt 84/11**

(84) Benannte Vertragsstaaten: **AT BE CH DE FR GB IT LI LU NL SE**

(56) Entgegenhaltungen:
**DE - A - 2 753 244**
**GB - A - 2 001 650**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen,** ***Müllerstrasse 170/178 Postfach 65 03 11,*** **D-1000 Berlin 65 (DE)**

(72) Erfinder: **Skuballa, Werner, Dr., Olwenstrasse 25, D-1000 Berlin 28 (DE)**
Erfinder: **Vorbrüggen, Helmut, Prof., Wilkestrasse 7, D-1000 Berlin 27 (DE)**
Erfinder: **Radüchel, Bernd, Dr., Gollanczstrasse 132, D-1000 Berlin 28 (DE)**
Erfinder: **Casals-Stenzel, Jorge, Dr., Wichernstrasse 20, D-1000 Berlin 33 (DE)**
Erfinder: **Schillinger, Ekkehard, Dr., Im Amseltal 50, D-1000 Berlin 28 (DE)**
Erfinder: **Town, Michael Harold, Dr., Buchsbaumweg 3, D-1000 Berlin 47 (DE)**

**EP 0 051 557 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

5-Cyan-prostacyclinderivate, Verfahren zu ihrer Herstellung und ihre Verwendung als Arzneimittel

Die Erfindung betrifft 5-Cyan-prostacyclinderivate, Verfahren zu ihrer Herstellung sowie ihre Verwendung als Arzneimittel.

Prostacyclin ($PGI_2$), eines der Hauptfaktoren bei der Blutplättchenaggregation, wirkt dilatierend auf verschiedene Blutgefäße (Science 196, 1072) und ist daher als Mittel zur Blutdrucksenkung anzusehen. $PGI_2$ besitzt jedoch nicht die für ein Arzneimittel notwendige Stabilität. So beträgt die Halbwertszeit von $PGI_2$ bei physiologischen pH-Werten und bei Raumtemperatur nur wenige Minuten. Aus der DE-OS 2 753 244 sind bereits Prostacyclinderivate bekannt, in denen die Stabilität durch einen Cyanrest an der Enolätherdoppelbindung erheblich gesteigert werden konnte.

Es wurde nun gefunden, daß sich Selektivität, Wirkungsdauer und Wirksamkeit dieser 5-Cyanoprostacycline durch Einführung einer Dreifachbindung in 18-Stellung gegebenenfalls mit zusätzlichen Alkylgruppen in der unteren Kette des 5-Cyanoprostacyclins noch weiter verbessern lassen.

Die erfindungsgemäßen Verbindungen wirken blutdrucksenkend und bronchodilatorisch. Sie sind außerdem zur Inhibierung der Thrombozytenaggregation geeignet.

Die Erfindung betrifft daher 5-Cyanprostacycline der allgemeinen Formel I

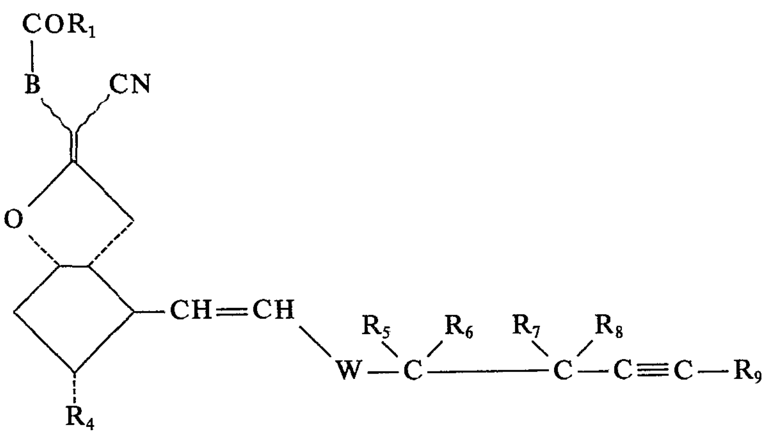

(I)

worin

$R_1$ den Rest $OR_2$, wobei $R_2$ Wasserstoff oder Alkyl mit 1–4 C-Atomen bedeutet, oder den Rest $NHR_3$ mit $R_3$ in der Bedeutung eines Methansulfonyl- oder Alkanoylrestes mit 1–10 C-Atomen oder eines Wasserstoffatoms,

B eine geradkettige oder verzweigtkettige Alkylengruppe mit 1–10 C-Atomen,

W eine freie oder funktionell abgewandelte Hydroxymethylengruppe oder eine freie oder funktionell abgewandelte

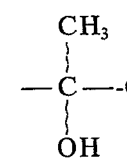

-Gruppe

wobei die OH-Gruppe $\alpha$- oder $\beta$-ständig sein kann,

$R_4$ eine freie oder funktionell abgewandelte Hydroxygruppe,

$R_5$, $R_6$, $R_7$, $R_8$ ein Wasserstoffatom oder eine Alkylgruppe mit 1–5 C-Atomen oder eine Methoxygruppe,

$R_9$ eine Alkylgruppe mit 1–5 C-Atomen und, falls

$R_2$ die Bedeutung eines Wasserstoffatoms hat, deren Salze mit physiologisch verträglichen Basen bedeuten.

Als Alkylgruppe $R_2$ sind gerade oder verzweigte Alkylgruppen mit 1–4 C-Atomen zu betrachten, wie beispielsweise Methyl, Äthyl, Propyl, Butyl, Isobutyl und tert.-Butyl.

Als Säurerest $R_3$ kommen der Methansulfonyl- oder ein Alkanoylrest mit 1–10 C-Atomen in Frage.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure und Caprinsäure.

Die Hydroxy ,pen $R_4$ und in W können funktionell abgewandelt sein, beispielsweise durch

Verätherung oder Veresterung, wobei die freien oder abgewandelten Hydroxygruppen in W $\alpha$- oder $\beta$-ständig sein können, und wobei freie Hydroxygruppen bevorzugt sind. Als Äther- und Acylreste kommen die dem Fachmann bekannten Reste in Betracht. Bevorzugt sind leicht abspaltbare Ätherreste, wie beispielsweise der Tetrahydropyranyl-, Tetrahydrofuranyl-, $\alpha$-Äthoxyäthyl-, Trimethylsilyl-, Dimethyl-tert.-butylsilyl- und Tribenzyl-silyl-rest. Als Acylreste kommen $C_1-C_4$-Alkanoylreste wie beispielsweise Acetyl, Propionyl, Butyryl oder Benzoyl in Frage.

Als Alkylengruppen B kommen geradkettige Alkylenreste mit 1—10, insbesondere 1—5 C-Atomen, in Frage. Beispielsweise seien genannt: Methylen, Äthylen, Trimethylen, Tetramethylen und Pentamethylen.

Als Alkylgruppen $R_5$, $R_6$, $R_7$, $R_8$, $R_9$ kommen gerad- und verzweigtkettige Alkylreste mit 1—5 Kohlenstoffatomen in Betracht, wie beispielsweise Methyl, Äthyl, Propyl, Isopropyl, Butyl, Isobutyl, tert.-Butyl, Pentyl und Neopentyl. Bevorzugte Reste sind Methyl und Äthyl.

Zur Salzbildung sind anorganische und organische Basen geeignet, wie sie dem Fachmann zur Bildung physiologisch verträglicher Salze bekannt sind. Beispielsweise genannt seien Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin und Tris-(hydroxymethyl)-methylamin.

Die Erfindung betrifft ferner ein Verfahren zur Herstellung der erfindungsgemäßen 5-Cyan-prostacyclinderivate der allgemeinen Formel I, dadurch gekennzeichnet, daß man eine Verbindung der allgemeinen Formel II

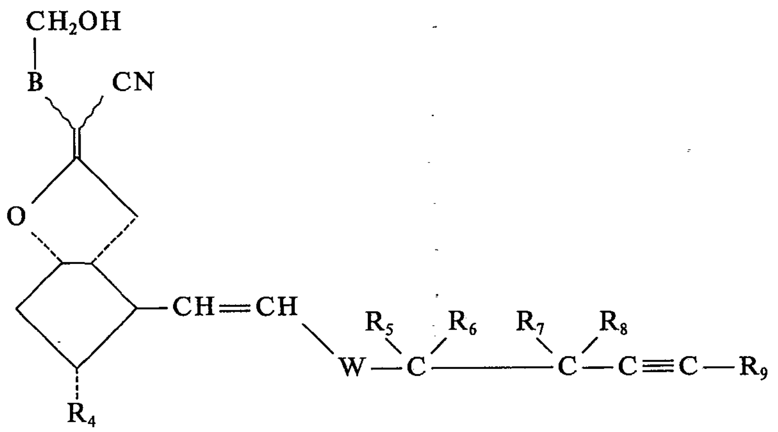

(II)

worin B, $R_5$, $R_6$, $R_7$, $R_8$ und $R_9$ die oben angegebenen Bedeutungen haben und freie Hydroxygruppen in $R_4$ und W geschützt sind, oxidiert und gegebenenfalls anschließend in beliebiger Reihenfolge Isomere trennt, geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe mit Verbindungen der allgemeinen Formel III

$$O=C=N-R_3 \qquad (III)$$

worin $R_3$ die oben angegebene Bedeutung hat, umsetzt oder eine Carboxylgruppe mit einer physiologisch verträglichen Base in ein Salz überführt.

Die Oxidation der 1-Hydroxygruppe wird nach den dem Fachmann bekannten Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff [Adv. in Carbohydrate Chem. 17, 169 (1962)].

Die Oxidation mit Pyridiniumdichromat wird bei Temperaturen von 0 bis 100° C, vorzugsweise 20 bis 40° C in einem gegen das Oxidationsmittel inerten Lösungsmittel, beispielsweise Dimethylformamid, durchgeführt.

Die Oxidation mit Jones-Reagenz wird bei Temperaturen von −40 bis +40° C, vorzugsweise 0 bis 30° C in Aceton als Lösungsmittel ausgeführt.

Die Oxidation mit Platin/Sauerstoff wird bei Temperaturen von 0 bis 60° C, vorzugsweise 20−40° C in einem gegen das Oxidationsmittel inerten Lösungsmittel, z. B. Essigester, durchgeführt.

Die Verseifung der Prostacyclinester wird nach den dem Fachmann bekannten Methoden durchgeführt, wie beispielsweise mit basischen Katalysatoren.

Die Einführung der Estergruppe $-OR_2$ für $R_1$, bei welcher $R_2$ eine Alkylgruppe mit 1−4 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der

Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Die für die Umsetzungen erforderlichen Diazoalkane sind entweder bekannt oder können nach bekannten Methoden hergestellt werden [Org. Reactions Bd. 8, Seiten 389–394 (1954)].

Die 5-Cyan-prostacyclinderivate der allgemeinen Formel I mit $R_1$ in der Bedeutung einer Hydroxygruppe können mit geeigneten Mengen der entsprechenden anorganischen Basen unter Neutralisierung in Salze überführt werden. Beispielsweise erhält man beim Lösen der entsprechenden Prostacyclinsäure in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die PG-Säure zum Beispiel in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen in $R_4$ oder W erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10fachen Menge des theoretischen Bedarfs. Die Umsetzung ist normalerweise bei 0–30° C nach 15–30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid oder Säureanhydrid, umsetzt.

Die Freisetzung einer funktionell abgewandelten OH-Gruppe zu den Verbindungen der allgemeinen Formel I erfolgt nach an sich bekannten Methoden. Beispielsweise wird die Abspaltung von Ätherschutzgruppen in einer wäßrigen Lösung einer organischen Säure, wie zum Beispiel Essigsäure oder Propionsäure oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 und 80° C durchgeführt.

Die Abspaltung der Silylätherschutzgruppen erfolgt beispielsweise mit Tetrabutylammoniumfluorid oder mit KF in Gegenwart eines Kronenäthers. Als Lösungsmittel sind beispielsweise geeignet Tetrahydrofuran, Diäthyläther, Dioxan oder Methylenchlorid. Die Abspaltung wird vorzugsweise bei Temperaturen zwischen 0 und 80° C durchgeführt.

Die Verseifung der Acylgruppen erfolgt beispielsweise mit Alkali- oder Erdalkalicarbonaten oder -hydroxyden in einem Alkohol oder der wäßrigen Lösung eines Alkohols. Als Alkohole kommen aliphatische Alkohole in Betracht, wie zum Beispiel Methanol, Äthanol oder Butanol, vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei −10 bis 70°C, vorzugsweise bei 25° C.

Die Umsetzung der Verbindung der allgemeinen Formel I mit $R_2$ in der Bedeutung eines Wasserstoffatoms mit einem Isocyanat der allgemeinen Formel III erfolgt gegebenenfalls unter Zusatz eines tertiären Amins, wie zum Beispiel Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Benzol, Toluol, Dimethylsulfoxid, bei Temperaturen ober- oder unterhalb Raumtemperatur, zum Beispiel zwischen −80 bis 100°C, vorzugsweise bei 0 bis 30° C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediär geschützt sind.

Die als Ausgangsmaterial dienenden Verbindungen der allgemeinen Formel II können beispielsweise hergestellt werden, indem man in an sich bekannter Weise einen Aldehyd der Formel IV [E. J. Corey et al. JACS 91, 5675 (1969); E. W. Yankee et al. JACS 96, 5865 (1974)]

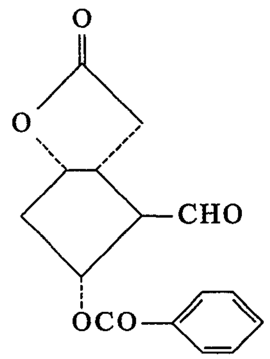

(IV)

mit einem Phosphonat der allgemeinen Formel V ,

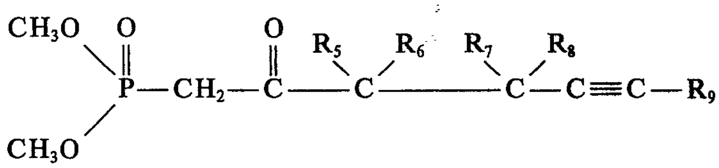

(V)

worin $R_5$, $R_6$, $R_7$, $R_8$ und $R_9$ die oben angegebene Bedeutung aufweisen, in einer Olefinierungsreaktion zu einem Keton der allgemeinen Formel VI umsetzt.

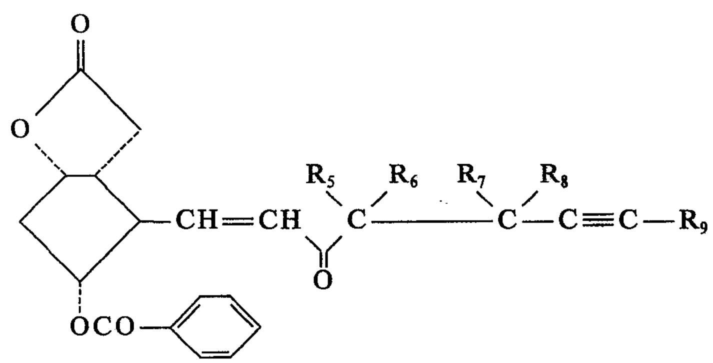

(VI)

Nach Reduktion der 15-Ketogruppe mit Zinkborhydrid oder Natriumborhydrid oder Umsetzung mit Alkylmagnesiumbromid oder Alkyllithium erhält man die epimeren 15$\alpha$- und 15$\beta$-Alkohole (PG-Numerierung), die gewünschtenfalls getrennt werden können und gegebenenfalls durch Veresterung oder Verätherung in die Verbindungen der allgemeinen Formel VII übergeführt werden.

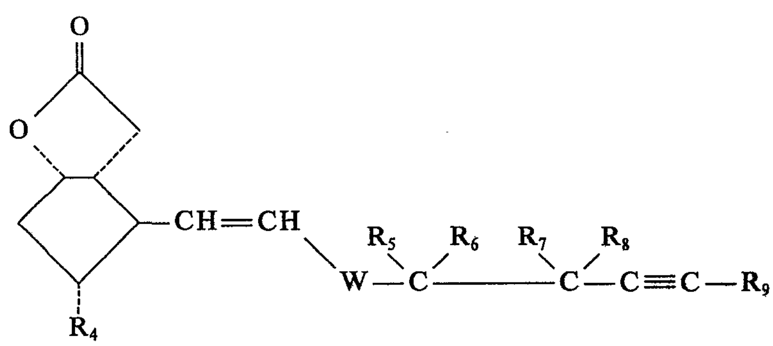

(VII)

Die Umsetzung des Lactons der allgemeinen Formel VII mit einem aus dem Nitril der allgemeinen Formel VIII und Lithiumdiisopropylamid hergestellten Carbanions

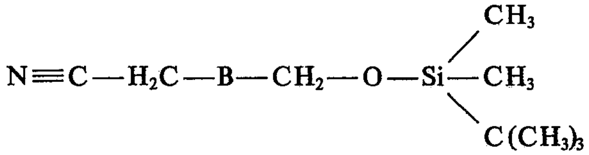

(VIII)

liefert nach Behandlung mit einer katalytischen Menge einer Säure, beispielsweise Bortrifluorid oder p-Toluolsulfonsäure, und anschließendem Schutz freier Hydroxygruppen durch Veresterung den Enoläther der allgemeinen Formel IX

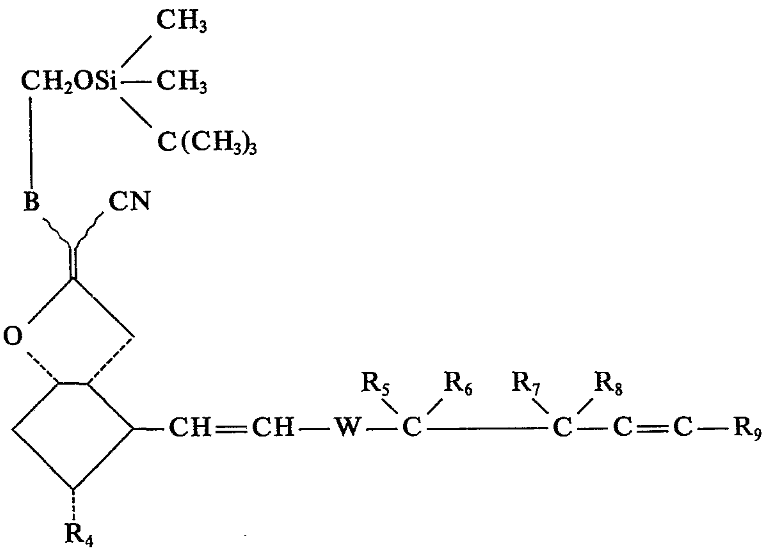

(IX)

Nach Abspaltung der Silylätherschutzgruppe gelangt man zu Verbindungen der allgemeinen Formel II. Je nach Bedeutung der funktionell abgewandelten Hydroxygruppen in W und $R_4$ erfolgt die Silylätherspaltung mit Tetrabutylammoniumfluorid oder mit einer wäßrigen Lösung einer organischen Säure, wie zum Beispiel Essigsäure.

Die durch die vorstehenden Reaktionen hergestellten an der Cyan-Doppelbindung isomeren Cyanoprostacycline können gegebenenfalls durch übliche Trennmethoden, wie beispielsweise Säulenchromatographie oder Schichtchromatographie getrennt werden.

Die Herstellung der Phosphonate der allgemeinen Formel V erfolgt in an sich bekannter Weise durch Umsetzung eines Alkylhalogenids der allgemeinen Formel X

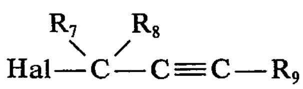

(X)

mit dem Dianion des Phosphonats der allgemeinen Formel XI

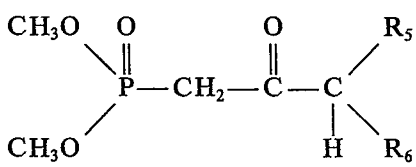

(XI)

Ein weiterer Zugang zu den Phosphonaten der allgemeinen Formel V besteht in der Umsetzung des Anions von Methylphosphonsäuredimethylester mit einem Ester der allgemeinen Formel XII

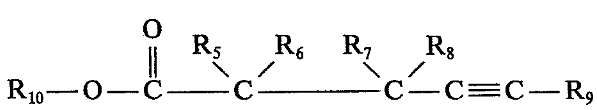

(XII)

worin $R_5$, $R_6$, $R_7$, $R_8$ und $R_9$ die oben angegebenen Bedeutungen aufweisen und $R_{10}$ eine Alkylgruppe mit 1–5 C-Atomen bedeutet, den man aus dem entsprechenden Malonsäureester durch Alkylierung mit dem Halogenid der allgemeinen Formel X und anschließender Decarbalkoxylierung erhalten kann.

Das für das obige Verfahren verwendete Nitril der allgemeinen Formel VIII kann beispielsweise aus 1,5-Pentandiol durch selektive Silylierung, Tosylierung und anschließende Umsetzung mit Kaliumcyanid hergestellt werden.

Die Verbindungen dieser Erfindung wirken blutdrucksenkend und bronchodilatorisch. Sie sind weiterhin geeignet zur Hemmung der Thrombozyten-Aggregation. Folglich stellen die neuen 5-Cyan-prostacyclinderivate der allgemeinen Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu P[illegible]ichnen sie sich durch größere Stabilität aus. Die hohe Gewebsspezifität der neuen

Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Prostaglandin-Analoga besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonaren vaskulären Widerstandes und des pulmonaren Blutdruckes, Förderung der Nierendurchblutung, Anwendung an Stelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose und Erhöhung der zerebralen Durchblutung. Außerdem besitzen die neuen Prostaglandin-Analoga antiprolifertive Eigenschaften und zytoprotektive Wirkung am Pankreas.

Die Dosis der Verbindungen ist 1 – 1500 µg/kg/Tag, wenn sie am menschlichen Patienten verabreicht werden. Die Einheitsdosis für den pharmazeutischen akzeptablen Träger beträgt 0,01 – 100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z. B. zur Herstellung von Blutdrucksenkern dienen.

## Beispiel 1

### 5-Cyano-18,19-tetradehydro-prostacyclin

Eine Lösung aus 325 mg 5-Cyano-2-descarboxy-2-hydroxymethyl-18,19-tetradehydro-prostacyclin-11,15-diacetat in 5 ml Dimethylformamid versetzt man mit 880 mg Pyridinium-dichromat und rührt die Mischung 29 Stunden bei Raumtemperatur. Anschließend verdünnt man mit 60 ml Wasser, extrahiert dreimal mit je 50 ml einer Mischung aus Äther/Pentan (2+1), schüttelt den organischen Extrakt dreimal mit je 20 ml Wasser, trocknet über Magnesiumsulfat und dampft im Vakuum ein. Nach Filtration des Rückstandes über Kieselgel erhält man mit Äther/Essigester (1+1) 240 mg 5-Cyano-18,19-tetradehydroprostacyclin-11,15-diacetat.

Man löst das Diacetat in 12 ml Methanol, versetzt mit 280 mg wasserfreiem Kaliumcarbonat und rührt 16 Stunden bei Raumtemperatur. Anschließend säuert man mit 10%iger Citronensäurelösung auf einen pH-Wert von 5 an, extrahiert mit Methylenchlorid, schüttelt den organischen Extrakt dreimal mit Sole und trocknet über Magnesiumsulfat. Den Eindampfrückstand chromatographiert man mit Methylenchlorid/Isopropanol (85+15) an Kieselgel. Dabei erhält man 170 mg der Titelverbindung als farbloses Öl.

IR: 3600, 3420 (breit), 2920, 2200, 1713, 1650, 970/cm.

Das Ausgangsmaterial für die obige Titelverbindung wird wie folgt hergestellt:

1a) (1S,5R,6R,7R)-6-[(E) – (3S)-3-Benzoyloxy-1-octen-6-inyl]-7-benzoyloxy-2-oxabicyclo[3.3.0]octan-3-on

Zu einer Lösung von 3,8 g (1S,5R,6R,7R)-6-[(E)-(3S)-3-Hydroxy-1-octen-6-inyl])7-benzoyloxy-2-oxabicyclo[3.3.0]octan-3-on (beschrieben in DE-OS 2 729 960) in 18 ml Pyridin fügt man bei Eisbadtemperatur 2,3 ml Benzoylchlorid und rührt 18 Stunden bei Raumtemperatur. Anschließend versetzt man mit 1 ml Wasser, rührt 2 Stunden, verdünnt die Mischung mit 250 ml Äther, schüttelt einmal mit Wasser, zweimal mit 10%iger Schwefelsäure, einmal mit 5%iger Natriumbicarbonatlösung und dreimal mit Wasser. Man trocknet über Magnesiumsulfat, dampft im Vakuum ein und

filtriert den Rückstand über Kieselgel. Mit Äther/Hexan (8+2) erhält man 4,6 g des Dibenzoats als farbloses Öl.
IR: 2970, 2923, 1770, 1715, 1602, 1585, 1270, 968/cm.

1b) 5-Cyano-2-descarboxy-2-(dimethyl-tert.-butylsilyloxymethyl)-18,19-tetradehydro-prostacyclin-11,15-diacetat

2,55 ml Diisopropylamin versetzt man bei −25° C innerhalb von 15 Minuten mit 11,8 ml einer 1,53-molaren Lösung von Butyllithium in Hexan und rührt 1 Stunde bei −25° C. Anschließend versetzt man mit 3,2 ml Hexamethyl-phosphorsäuretriamid und tropft in diese Mischung bei −70° C innerhalb von 30 Minuten eine Lösung aus 4,1 g 6-(Dimethyl-tert.-butylsilyloxy)-hexannitril in 4 ml Tetrahydrofuran. Man rührt 20 Minuten bei −70° C, fügt eine Lösung von 2,4 g des nach Beispiel 1a hergestellten Dibenzoats in 15 ml Äther und 15 ml Tetrahydrofuran zu, rührt 20 Minuten und säuert die Reaktionsmischung durch Eingießen in eine 10%ige Citronensäurelösung auf einen pH-Wert von 5 an. Man extrahiert mit Äther, wäscht die organische Phase mit Wasser neutral, trocknet über Magnesiumsulfat und filtriert den Eindampfrückstand mit Essigester über Kieselgel. Dabei erhält man 1,7 g des Reaktionsproduktes der metallorganischen Umsetzung als 11,15-Dihydroxyverbindung.
Zur Wasserabspaltung löst man das Reaktionsprodukt der vorstehend beschriebenen Umsetzung in 80 ml abs. Äther, fügt 50 ml einer verdünnten ätherischen Bortrifluorid-Lösung (Herstellung: Man verdünnt 0,5 ml 45%ige Bortrifluorid-ätherat-Lösung mit 45 ml abs. Äther) zu und rührt 1 Stunde bei Raumtemperatur. Anschließend gießt man auf 5%ige Natriumbicarbonatlösung, wäscht mit Wasser neutral, trocknet über Magnesiumsulfat und dampft im Vakuum ein.
Den Rückstand löst man in 6 ml Pyridin, fügt 2 ml Essigsäureanhydrid zu und rührt 16 Stunden bei Raumtemperatur. Anschließend dampft man im Vakuum ein und chromatographiert den Rückstand an Kieselgel. Mit Hexan/Äther (3+2) erhält man zunächst 720 mg der Titelverbindung (5E-konfiguriert) und als polarere Komponente 610 mg des isomeren (5Z)-5-Cyano-2-descarboxy-2-(dimethyl-tert.-butylsilyloxymethyl)-18,19-tetradehydro-prostacyclin-11,15-diacetats.
IR: 2953, 2930, 2860, 2200, 1732, 1651, 1240, 970, 835/cm.

1c) 5-Cyano-2-descarboxy-2-hydroxymethyl-18,19-tetradehydro-prostacyclin-11,15-diacetat

0,7 g des nach Beispiel 1b hergestellten Diacetats rührt man mit 20 ml einer Mischung aus Eisessig/Wasser/Tetrahydrofuran (65+35+10) 16 Stunden bei Raumtemperatur und dampft anschließend im Vakuum ein. Nach Chromatographie des Rückstandes an Kieselgel erhält man mit Äther/Essigester (1+1) 530 mg der Titelverbindung als farbloses Öl.
IR: 3610, 2960, 2200, 1732, 1650, 1240, 970/cm.

1d) 6-(Dimethyl-tert.-butylsilyloxy)-hexannitril

Zu einer Lösung von 62,5 g 1,5-Pentandiol und 102 g Imidazol in 100 ml Dimethylformamid fügt man bei Eisbadtemperatur 90,5 g Dimethyl-tert.-butylsilylchlorid und rührt 16 Stunden bei 0° C. Anschließend gießt man auf 900 ml Wasser, extrahiert dreimal mit je 500 ml einer Mischung aus Hexan/Äther (1+1), wäscht den organischen Extrakt mit Wasser neutral und trocknet über Magnesiumsulfat. Man engt im Vakuum ein und destilliert den Rückstand im Vakuum bei 0,6 Torr. Dabei erhält man bei 76−80° C 55 g des Monosilyläthers als farblose Flüssigkeit.
Zur Tosylierung löst man in 185 ml Pyridin, fügt 74 g p-Toluolsulfonsäurechlorid zu und rührt 16 Stunden bei Raumtemperatur. Anschließend versetzt man mit 10 ml Wasser, rührt 3 Stunden, verdünnt mit 1,3 Ltr. Äther, schüttelt nacheinander mit 10%iger Schwefelsäure, mit Wasser, mit 5%iger Natriumbicarbonatlösung und mit Wasser. Man trocknet über Magnesiumsulfat und dampft im Vakuum ein.
Den Rückstand löst man in 185 ml Dimethylsulfoxid, fügt 22 g Natriumcyanid zu und rührt 18 Stunden bei 80° C unter Argon. Anschließend versetzt man mit 700 ml Wasser, extrahiert dreimal mit je 400 ml einer Mischung aus Äther/Hexan (1+1), wäscht den organischen Extrakt mit Wasser neutral und trocknet über Magnesiumsulfat. Man engt im Vakuum ein und destilliert den Rückstand im Vakuum bei 0,1 Torr. Dabei erhält man bei 75−77° C 43 g der Titelverbindung als farblose Flüssigkeit.
IR: 2930, 2855, 2242, 1250, 1095, 830/cm.

Beispiel 2

5-Cyano-(16RS)-16-methyl-18,19-tetradehydro-prostacyclin

Eine Mischung aus 670 mg 5-Cyano-2-descarboxy-2-hydroxymethyl-(16RS)-16-methyl-18,19-tetradehydro-prostacyclin-11,15-diacetat, 1,8 g Pyridinium-dichromat und 10 ml Dimethylformamid rührt man 30 Stunden bei Raumtemperatur. Anschließend verdünnt man mit Wasser, extrahiert mit einer Mischung aus Äther/Pentan (2+1), wäscht den organischen Extrakt mit Wasser, trocknet über Magnesiumsulfat und dampft im Vakuum ein. Nach Filtration des Rückstandes über Kieselgel erhält man mit Äther/Essigester (1+1) 460 mg 5-Cyano-(16RS)-16-methyl-18,19-tetradehydro-prostacyclin-11,15-diacetat.

Man rührt das Diacetat 16 Stunden bei Raumtemperatur in 22 ml Methanol mit 550 mg Kaliumcarbonat (wasserfrei). Anschließend säuert man mit 10%iger Citronensäurelösung auf einen pH-Wert von 5 an, extrahiert mit Methylenchlorid, schüttelt den organischen Extrakt dreimal mit Sole und trocknet über Magnesiumsulfat. Den Eindampfrückstand chromatographiert man mit Methylenchlorid/Isopropanol (85+15) an Kieselgel. Dabei erhält man 220 mg der Titelverbindung als Öl.

IR: 3610, 3400 (breit), 2925, 2200, 1712, 1650, 972/cm.

Das Ausgangsmaterial für die obige Titelverbindung wird wie folgt hergestellt:

2a) (1S,5R,6R,7R)-6-[(E)-(3S,4RS)-Benzoyloxy-4-methyl-1-octen-6-in-yl)-7-benzoyloxy-2-oxabicyclo[3.3.0]octan-3-on

In Analogie zu Beispiel 1a erhält man aus 4,1 g (1S,5R,6R,7R)-6-[(E)-(3S,4RS)-3-Hydroxy-4-methyl-1-octen-6-inyl]-7-benzoyloxy-2-oxabicyclo[3.3.0]octan-3-on 4,9 g der Titelverbindung als farbloses Öl.

IR: 2965, 2920, 1770, 1715, 1602, 1585, 1450, 1315, 1270, 1110, 969/cm.

2b) 5-Cyano-2-descarboxy-2-(dimethyl-tert.-butylsilyloxymethyl)-(16RS)-16-methyl-18,19-tetradehydro-prostacyclin-11,15-diacetat

Man tropft bei −25°C 12 ml einer 1,5molaren Lösung von Butyllithium in Hexan zu 2,6 ml Diisopropylamin, rührt 1 Stunde und versetzt dann mit 3,3 ml Hexamethylphosphorsäuretriamid. In diese Mischung tropft man bei −70°C eine Lösung aus 4,2 g 6-(Dimethyl-tert.-butylsilyloxy)-hexannitril in 5 ml Tetrahydrofuran, rührt 20 Minuten bei −70°C und fügt eine Lösung von 2,5 g des nach Beispiel 2a hergestellten Dibenzoats in 15 ml Äther und 15 ml Tetrahydrofuran zu. Nach 20 Minuten säuert man die Reaktionsmischung durch Eingießen in eine 10%ige Citronensäurelösung auf einen pH-Wert von 5 an, extrahiert mit Äther, schüttelt den organischen Extrakt mit Wasser, trocknet über Magnesiumsulfat und dampft im Vakuum ein. Den Rückstand filtriert man mit Essigester über Kieselgel. Dabei erhält man 1,6 g des Reaktionsproduktes der metallorganischen Umsetzung als 11,15-Diol, das man in 80 ml Äther löst und mit 50 ml einer verdünnten ätherischen Bortrifluoridlösung (Herstellung Beispiel 1b) 1 Stunde bei Raumtemperatur rührt. Anschließend gießt man auf 5%ige Natriumbicarbonatlösung, wäscht mit Wasser neutral, trocknet über Magnesiumsulfat und dampft im Vakuum ein.

Den Rückstand rührt man 16 Stunden bei Raumtemperatur mit 6 ml Pyridin und 2 ml Essigsäureanhydrid. Anschließend dampft man im Vakuum ein und chromatographiert den Rückstand an Kieselgel. Mit Hexan/Äther (3+2) erhält man zunächst 690 mg der Titelverbindung (5E-konfiguriert) und als polarere Komponente 580 mg des isomeren (5Z)-5-Cyano-2-descarboxy-2-(dimethyl-tert.-butylsilyloxymethyl)-(16RS)-16-methyl-18,19-tetrahydro-prostacyclin-11,15,diacetates.

IR: 2955, 2935, 2860, 2200, 1731, 1650, 1240, 970, 835/cm.

2c) 5-Cyano-2-descarboxy-2-hydroxymethyl-(16RS)-16-methyl-18,19-tetradehydro-prostacyclin-11,15-diacetat

In Analogie zu Beispiel 1c erhält man aus 0,6 g des nach Beispiel 2b hergestellten Diacetats 480 mg der Titelverbindung als farbloses Öl.

IR: 3600, 3400, 2965, 2860, 2200, 1730, 1651, 1240, 972/cm.

Beispiel 3

(5Z)-5-Cyano-(16RS)-16-methyl-18,19-tetradehydro-prostacyclin

Eine Mischung aus 340 mg (5Z)-5-Cyano-2-descarboxy-2-hydroxymethyl-(16RS)-16-methyl-18,19-tetradehydro-prostacyclin-11,15-diacetat, 950 mg Pyridinium-dichromat und 5 ml Dimethylformamid rührt man 28 Stunden bei Raumtemperatur. Anschließend verdünnt man mit Wasser, extrahiert mit einer Mischung aus Äther/Pentan (2+1), schüttelt den organischen Extrakt mit Wasser, trocknet über Magnesiumsulfat und dampft im Vakuum ein. Nach Filtration des Rückstandes über Kieselgel erhält man mit Äther/Essigester (1+1) 220 mg (5Z)-5-Cyano-(16RS)-16-methyl-18,19-tetradehydro-prostacyclin-11,15-diacetat. Man rührt das Diacetat 16 Stunden bei Raumtemperatur in 10 ml Methanol mit 280 mg Kaliumcarbonat (wasserfrei), säuert anschließend mit 10%iger Citronensäure an, extrahiert mit Methylenchlorid, schüttelt den organischen Extrakt mit Wasser und trocknet über Magnesiumsulfat. Nach Chromatographie des Eindampfrückstandes an Kieselgel erhält man mit Methylenchlorid/Isopropanol (85+15) 105 mg der Titelverbindung als Öl.
IR: 3600, 3400, 2925, 2200, 1710, 1654, 972/cm.

Das Ausgangsmaterial für die obige Titelverbindung wird wie folgt hergestellt:

3a) (5Z)-5-Cyano-2-descarboxy-2-hydroxymethyl-(16RS)-16-methyl-18,19-tetradehydro-prostacyclin-11,15-diacetat

0,55 g (5Z)-5-Cyano-2-descarboxy-2-(dimethyl-tert.-butylsilyloxymethyl)-(16RS)-16-methyl-18,19-tetradehydro-prostacyclin-11,15-diacetat rührt man mit 16 ml einer Mischung aus Eisessig/Wasser/Tetrahydrofuran (65+35+10) 16 Stunden bei Raumtemperatur und dampft anschließend im Vakuum ein. Nach Chromatographie des Rückstandes an Kieselgel erhält man mit Äther/Essigester (1+1) 400 mg der Titelverbindung als farbloses Öl.
IR: 3600, 3410, 2962, 2860, 2200, 1734, 1653, 1240, 970/cm.

Beispiel 4

5-Cyano-16,16-dimethyl-18,19-tetradehydro-prostacyclin

In Analogie zu Beispiel 1 erhält man aus 0,48 g 5-Cyano-2-descarboxy-2-hydroxymethyl-16,16-dimethyl-18,19-tetradehydro-prostacyclin-11,15-diacetat 230 mg der Titelverbindung als farbloses Öl.
IR: 3600, 3400, 2925, 2200, 1712, 1650, 973/cm.

Das Ausgangsmaterial für die obige Titelverbindung wird wie folgt hergestellt:

4a) (1S,5R,6R,7R)-6-[(E)-(3R)-3-Benzoyloxy-4,4-dimethyl-1-octen-6-inyl]-7-benzoyloxy-2-oxabicyclo[3.3.0]octan-3-on

In Analogie zu Beispiel 1a erhält man aus 1,5 g (1S,5R,6R,7R)-6-[(E)-(3R)-3-Hydroxy-4,4-dimethyl-1-octen-6-inyl]-7-benzoyloxy-2-oxabicyclo[3.3.0]octan-3-on, 6 ml Pyridin und 1,2 ml Benzoylchlorid 1,85 g der Titelverbindung als Öl.
IR: 2965, 2925, 1770, 1715, 1600, 1586, 1270, 970/cm.

4b) 5-Cyano-2-descarboxy-2-(dimethyl-tert.-butylsilyloxymethyl)-16,16-dimethyl-18,19-tetradehydro-prostacyclin-11,15-diacetat

In Analogie zu Beispiel 1b erhält man aus 1,3 g des nach Beispiel 4a hergestellten Dibenzoats 320 mg der Titelverbindung als farbloses Öl.
IR: 2955, 2925, 2200, 1733, 1650, 1240, 972, 835/cm.

4c) 5-Cyano-2-descarboxy-2-hydroxymethyl-16,16-dimethyl-18,19-tetradehydro-prostacyclin-11,15-diacetat

In Analogie zu Beispiel 1c erhält man aus 0,3 g des nach Beispiel 4b hergestellten Diacetats 205 mg der Titelverbindung als farbloses Öl.
IR: 3600, 3400, 2960, 2862, 2200, 1732, 1650, 972/cm.

Beispiel 5

5-Cyano-(15RS)-15-methyl-18,19-tetradehydro-prostacyclin

In Analogie zu Beispiel 1 erhält man aus 0,7 g 5-Cyano-2-descarboxy-2-hydroxymethyl-(15RS)-15-methyl-18,19-tetradehydro-prostacyclin-11-acetat 370 mg der Titelverbindung als farbloses Öl.
IR: 3610, 3400, 2920, 2200, 1712, 1650, 972/cm.
Das Ausgangsmaterial für die obige Titelverbindung wird wie folgt hergestellt:

5a) 5-Cyano-2-descarboxy-2-(dimethyl-tert.-butylsilyloxymethyl)-(15RS)-15-methyl-18,19-tetradehydro-prostacyclin-11-acetat

In Analogie zu Beispiel 1b erhält man aus 4,7 g (1S,5R,6R,7R)-6-[(E)-(3RS)-3-hydroxy-3-methyl-1-octen-6-inyl]-7-benzoyloxy-2-oxabicyclo[3.3.0]octan-3-on und 8 g 6-(Dimethyl-tert.-butylsilyloxy)-hexannitril 920 mg der Titelverbindung als Öl.
IR: 2950, 2925, 2860, 2200, 1733, 1650, 1240, 972, 835/cm.

5b) 5-Cyano-2-descarboxy-2-hydroxymethyl-(15RS)-15-methyl-18,19-tetradehydro-prostacyclin-11-acetat

In Analogie zu Beispiel 1c erhält man aus 0,8 g des nach Beispiel 5a hergestellten Acetats 0,6 g der Titelverbindung als farbloses Öl.
IR: 3600, 3400, 2960, 2860, 2200, 1732, 1651, 1240, 972/cm.

Beispiel 6

5-Cyano-(16RS)-16,20-dimethyl-18,19-tetradehydro-prostacyclin

Eine Mischung aus 0,7 g 5-Cyano-2-descarboxy-2-hydroxymethyl-(16RS)-16,20-dimethyl-18,19-tetradehydro-prostacyclin-11,15-diacetat, 1,9 g Pyridinium-dichromat und 10 ml Dimethylformamid rührt man 30 Stunden bei Raumtemperatur. Anschließend verdünnt man mit Wasser, extrahiert mit einer Mischung aus Äther/Pentan (2+1), wäscht den organischen Extrakt mit Wasser, trocknet über Magnesiumsulfat und dampft im Vakuum ein. Nach Filtration des Rückstandes über Kieselgel erhält man mit Äther/Essigester (1+1) 0,5 g 5-Cyano-(16RS)-16,20-dimethyl-18,19-tetradehydro-prostacyclin-11,15-diacetat. Man rührt das Diacetat 16 Stunden bei Raumtemperatur in 24 ml Methanol mit 0,6 g Kaliumcarbonat. Anschließend säuert man mit 10%iger Citronensäure auf einen pH-Wert von 5 an, extrahiert mit Methylenchlorid, schüttelt den organischen Extrakt mit Sole und trocknet über Magnesiumsulfat. Den Eindampfrückstand chromatographiert man mit Methylenchlorid/Isopropanol (85+5) an Kieselgel. Dabei erhält man 310 mg der Titelverbindung als farbloses Öl.
IR: 3600, 3400, 2925, 2200, 1713, 1651, 973/cm.
Das Ausgangsmaterial für die obige Titelverbindung wird wie folgt hergestellt:

6a) (1S,5R,6R,7R)-6-[(E)-(3S,4RS)-3-Benzoyloxy-4-methyl-1-nonen-6-inyl]-7-benzoyloxy-2-oxabicyclo[3.3.0]octan-3-on

In Analogie zu Beispiel 1a erhält man aus 3,5 g (1S,5R,6R,7R)-6-[(E)-(3S,4RS)-3-Hydroxy-4-methyl-1-nonen-6-inyl]-7-benzoyloxy-2-oxabicyclo[3.3.0]octan-3-on 4 g der Titelverbindung als Öl.
IR: 2960, 2925, 1771, 1715, 1602, 1586, 1450, 1315, 1270, 1100, 970/cm.

6b) 5-Cyano-2-descarboxy-2-(dimethyl-tert.-butylsilyloxymethyl)-(16RS)-16,20-dimethyl-18,19-tetradehydro-prostacyclin-11,15-diacetat

Zu 5,2 ml Diisopropylamin tropft man bei −25°C 24,2 ml einer 1,5molaren Butyllithiumlösung (in Hexan), rührt 1 Stunde und versetzt dann mit 7 ml Hexamethylphosphorsäuretriamid. In diese Mischung tropft man bei −70°C eine Lösung aus 8,5 g 6-(Dimethyl-tert.-butylsilyloxy)-hexannitril in 10 ml Tetrahydrofuran, rührt 20 Minuten bei −70°C und versetzt mit einer Lösung aus 5 g des nach Beispiel 6a hergestellten Dibenzoats in 30 ml Äther und 30 ml Tetrahydrofuran. Nach 20 Minuten säuert man die Reaktionsmischung durch Eingießen in eine 10%ige Citronensäurelösung auf einen pH-Wert von 5 an, extrahiert mit Äther, schüttelt den organischen Extrakt mit Wasser, trocknet über Magnesiumsulfat und dampft im Vakuum ein. Den Rückstand filtriert man mit

Essigester über Kieselgel. Dabei erhält man 3,3 g des Reaktionsproduktes der metallorganischen Reaktion als 11,15-Diol, das man in 200 ml Äther löst und mit 120 ml einer verdünnten ätherischen Bortrifluoridlösung (Herstellung: Beispiel 1b) 1 Stunde bei Raumtemperatur rührt. Anschließend gießt man auf 5%ige Natriumbicarbonatlösung, wäscht mit Wasser neutral, trocknet über Magnesiumsulfat und dampft im Vakuum ein. Den Rückstand rührt man 16 Stunden bei Raumtemperatur mit 12 ml Pyridin und 4 ml Essigsäureanhydrid. Anschließend dampft man im Vakuum ein und chromatographiert den Rückstand an Kieselgel. Mit Hexan/Äther (3+2) erhält man zunächst 1,4 g der Titelverbindung (5E-konfiguriert) und als polarere Komponente 1,2 g des isomeren (5Z)-5-Cyano-2-descarboxy-2-(dimethyl-tert.-butylsilyloxymethyl)-(16RS)-16,20-dimethyl-18,19-tetradehydro-prostacyclin-11,15-diacetats.
IR: 2960, 2935, 2862, 2200, 1732, 1650, 1240, 972, 835/cm.

6c) 5-Cyano-2-descarboxy-2-hydroxymethyl-(16RS)-16,20-dimethyl-18,19-tetradehydro-prostacyclin-11,15-diacetat

In Analogie zu Beispiel 1c erhält man aus 1,3 g des nach Beispiel 6b hergestellten Diacetats 1 g der Titelverbindung als Öl.
IR: 3600, 3410, 2963, 2860, 2200, 1730, 1650, 1240, 972/cm.

6d) 3-Methyl-2-oxo-oct-5-inyl-phosphonsäure-dimethylester

Zu einer Lösung von 15,8 g Natrium in 344 ml Äthylalkohol tropft man bei Raumtemperatur 120 g Methylmalonsäurediäthylester. Nach 30 Minuten tropft man 118 g 1-Brom-2-pentin (hergestellt aus Pent-2-in-1-ol mit Phosphortribromid in Pyridin) zu und erhitzt 16 Stunden unter Rückfluß. Anschließend filtriert man die Reaktionsmischung, wäscht mit Methylenchlorid und engt im Vakuum ein. Den Rückstand löst man in 500 ml Methylenchlorid, schüttelt zweimal mit je 50 ml Wasser, trocknet mit Magnesiumsulfat und engt im Vakuum ein. Den Rückstand destilliert man im Vakuum bei 12 Torr. Bei 135° C erhält man 147 g des alkylierten Methylmalonsäureesters, den man in 1,2 Ltr. Dimethylsulfoxid und 11 ml Wasser mit 51,6 g Lithiumchlorid 4,5 Stunden unter Rückfluß erhitzt. Anschließend gießt man auf 4,5 Ltr. Eiswasser, extrahiert mit Äther, schüttelt den organischen Extrakt zweimal mit Wasser, trocknet über Magnesiumsulfat und engt im Vakuum ein. Die Destillation des Rückstandes liefert bei 84° C und 12 Torr 82,5 g 2-Methyl-hept-4-insäureäthylester als farblose Flüssigkeit.
Zu einer Lösung von 176 g Methanphosphonsäuredimethylester in 2 Ltr. Tetrahydrofuran tropft man 646 ml einer 1,52%igen Butyllithiumlösung in Hexan bei −70° C, rührt 15 Minuten und fügt langsam eine Lösung von 82,5 g 2-Methyl-hept-4-insäureäthylester in 320 ml Tetrahydrofuran zu. Man rührt 4 Stunden bei −70° C, neutralisiert mit Essigsäure und dampft im Vakuum ein. Man versetzt den Rückstand mit 200 ml Wasser, extrahiert dreimal mit je 600 ml Methylenchlorid, schüttelt den organischen Extrakt einmal mit 100 ml Wasser, trocknet über Magnesiumsulfat und engt im Vakuum ein. Die Destillation des Rückstandes im Vakuum bei 0,6 Torr und 120° C liefert 85 g der Titelverbindung als farblose Flüssigkeit.

6e) (1S,5R,6R,7R)-6-[(E)-(4RS)-3-Oxo-4-methyl-1-nonen-6-inyl]-7-benzoyloxy-2-oxabicyclo[3.3.0]-octan-3-on

Zu einer Suspension von 1,4 g Natriumhydrid (50%ige Suspension in Öl) in 170 ml Dimethoxyäthan tropft man bei Raumtemperatur eine Lösung von 8,2 g des nach Beispiel 6d hergestellten Phosphonats in 30 ml Dimethoxyäthan und rührt 2 Stunden bei Raumtemperatur unter Argon. Anschließend versetzt man bei −20° C mit einer Lösung aus 7,6 g (1S,5R,6R,7R)-6-Formyl-7-benzoyloxy-2-oxabicyclo[3.3.0]octan-3-on in 80 ml Dimethoxyäthan, rührt 2 Stunden bei −20° C, neutralisiert mit Essigsäure, verdünnt mit Äther, schüttelt mit 4%iger Natriumbicarbonatlösung und Wasser, trocknet über Magnesiumsulfat und dampft im Vakuum ein. Nach Chromatographie des Rückstandes an Kieselgel erhält man mit Äther/Hexan (6+4) 8,2 g der Titelverbindung als farbloses Öl.
IR: 2962, 2230, 1772, 1718, 1690, 1625, 1600, 975/cm.

6f) (1S,5R,6R,7R)-6-[(E)-(3S,4RS)-3-Hydroxy-4-methyl-1-nonen-6-inyl]-benzoyloxy-2-oxabicyclo[3.3.0]octan-3-on

Zu einer Lösung von 6 g des nach Beispiel 6e hergestellten Ketons in 200 ml Methanol fügt man bei −40° C p ‧nsweise 3,4 g Natriumborhydrid und rührt 1 Stunde bei −40° C unter Argon.

Anschließend verdünnt man mit Äther, wäscht mit Wasser neutral, trocknet über Magnesiumsulfat und dampft im Vakuum ein. Durch Säulenchromatographie an Kieselgel mit Äther/Hexan (8+2) eluiert man zunächst 2,6 g der Titelverbindung (3α-Hydroxy) sowie als polarere Komponente 1,9 g der entsprechenden isomeren 3β-Hydroxyverbindung.
IR: (α-Alkohol): 3600, 3500, 2965, 1772, 1718, 1603, 1275, 975/cm.

## Beispiel 7

### (5Z)-5-Cyano-(16RS)-16,20-dimethyl-18,19-tetradehydro-prostacyclin

In Analogie zu Beispiel 3 erhält man aus 165 mg (5Z)-5-Cyano-2-descarboxy-2-hydroxymethyl-(16RS)-16,20-dimethyl-18,19-tetradehydro-prostacyclin-11,15-diacetat 65 mg der Titelverbindung als farbloses Öl.
IR: 3610, 3400, 2930, 2200, 1710, 1654, 974/cm.

Das Ausgangsmaterial für die obige Titelverbindung wird wie folgt hergestellt:

7b) (5Z)-5-Cyano-2-descarboxy-2-hydroxymethyl-(16RS)-16,20-dimethyl-18,19-tetradehydro-prostacyclin-11,15-diacetat

In Analogie zu Beispiel 3a erhält man aus 0,4 g (5Z)-5-Cyano-2-descarboxy-2-(dimethyl-tert.-butylsilyloxymethyl)-(16RS)-16,20-dimethyl-18,19-tetradehydro-prostacyclin-11,15-diacetat 0,29 g der Titelverbindung als Öl.

## Beispiel 8

### 5-Cyano-(16RS)-16-methyl-18,19-tetradehydro-prostacyclin-methylester

Man versetzt eine Lösung von 100 mg der nach Beispiel 2 hergestellten Säure in 10 ml Methylenchlorid bei 0° C tropfenweise mit einer ätherischen Diazomethanlösung bis zur bleibenden Gelbfärbung. Nach Eindampfen der Lösung im Vakuum filtriert man den Rückstand mit Methylenchlorid über wenig Kieselgel und erhält 87 mg des Methylesters als farbloses Öl.
IR: 3600, 3410, 2965, 2200, 1732, 1650, 972/cm.

## Beispiel 9

### 5-Cyano-(16RS)-16,20-dimethyl-18,19-tetradehydro-prostacyclin-methylester

In Analogie zu Beispiel 8 erhält man aus 120 mg der nach Beispiel 6 hergestellten Säure 110 mg der Titelverbindung als farbloses Öl.
IR: 3610, 3400, 2960, 2200, 1732, 1652, 974/cm.

## Beispiel 10

### 5-Cyano-(16RS)-16-methyl-18,19-tetradehydro-N-methansulfonyl-prostacyclin-carboxamid

Zu einer Lösung von 200 mg 5-Cyano-(16RS)-16-methyl-18,19-tetradehydro-prostacyclin-11,15-diacetat (hergestellt in Beispiel 2) in 6 ml Acetonitril fügt man 60 mg Triäthylamin und versetzt mit einer Lösung aus 75 mg Methylsulfonylisocyanat in 4 ml Acetonitril. Man rührt 4 Stunden bei 20° C, engt im Vakuum ein, versetzt mit 10 ml Wasser, neutralisiert mit 10%iger Citronensäurelösung und extrahiert mit Äther. Man schüttelt den organischen Extrakt mit Sole, trocknet über Magnesiumsulfat und dampft im Vakuum ein. Den Rückstand reinigt man durch präparative Schichtchromatographie mit Methylenchlorid/Isopropanol (99+1) und erhält 172 mg des Methansulfonylcarboxamids.

Zur Abspaltung der Acetatschutzgruppen löst man in 10 ml Methanol, versetzt mit 120 mg Kaliumcarbonat und rührt 3 Stunden bei 20° C unter Argon. Anschließend verdünnt man mit Sole, stellt die Mischung mit 1%iger Citronensäurelösung auf einen pH-Wert von 7 ein, extrahiert mit Methylenchlorid, schüttelt den Extrakt mit Sole, trocknet über Magnesiumsulfat und dampft im Vakuum ein. Nach Filtration über Kieselgel mit Methylenchlorid/Isopropanol (9+1) erhält man 85 mg der Titelverbindung als Öl.
IR: 3600, 3400, 2935, 2865, 2200, 1720, 1651, 1340, 975/cm.

## Beispiel 11

### 5-Cyano-(16RS)-16,20-dimethyl-18,19-tetradehydro-N-methansulfonyl-prostacyclin-carboxamid

In Analogie zu Beispiel 10 erhält man aus 280 mg 5-Cyano-(16RS)-16,20-dimethyl-18,19-tetradehydro-prostacyclin-11,15-diacetat 106 mg der Titelverbindung als farbloses Öl.
IR: 3600, 3410, 2935, 2864, 2200, 1720, 1651, 1340, 974/cm.

## Beispiel 12

### 5-Cyano-(16RS)-16-methyl-18,19-tetradehydro-N-acetyl-prostacyclin-carboxamid

Zu einer Lösung aus 200 mg 5-Cyano-(16RS)-16-methyl-18,19-tetradehydro-prostacyclin-11,15-diacetat in 6 ml Acetonitril fügt man eine Lösung aus 75 mg Triäthylamin in 6 ml Acetonitril und versetzt bei 0°C mit einer Lösung aus 55 mg Acetylisocyanat in 5 ml Acetonitril. Man rührt 2 Stunden bei Raumtemperatur, engt im Vakuum ein, versetzt mit 10 ml Wasser, stellt mit 1%iger Citronensäurelösung auf pH 7 ein und extrahiert mit Äther. Man schüttelt den Extrakt mit Sole, trocknet über Magnesiumsulfat und dampft im Vakuum ein. Nach Reinigung durch präparative Schichtchromatographie (Äther/Hexan 8 + 2) erhält man 155 mg des Acetylcarboxamids.

Zur Abspaltung der Acetatgruppen löst man in 5 ml Methanol, versetzt mit 100 mg Kaliumcarbonat und rührt 3 Stunden bei 20°C unter Argon. Anschließend verdünnt man mit Sole, stellt die Mischung mit 1%iger Citronensäurelösung auf pH 7 ein, extrahiert mit Methylenchlorid, schüttelt den Extrakt mit Sole, trocknet über Magnesiumsulfat und dampft im Vakuum ein. Nach Chromatographie an Kieselgel erhält man mit Methylenchlorid/Isopropanol (9 + 1) 100 mg der Titelverbindung als Öl.
IR: 3600, 3400, 2960, 2200, 1731, 1705, 1650, 972/cm.

## Beispiel 13

### 5-Cyano-(16RS)-16,20-dimethyl-18,19-tetradehydro-N-acetyl-prostacyclin-carboxamid

In Analogie zu Beispiel 12 erhält man aus 170 mg 5-Cyano-(16RS)-16,20-dimethyl-18,19-tetradehydro-prostacyclin-11,15-diacetat (hergestellt in Beispiel 6) 85 mg der Titelverbindung als farbloses Öl.
IR: 3610, 3400, 2960, 2200, 1732, 1706, 1651, 974/cm.

## Beispiel 14

### Tris-(hydroxymethyl)-aminomethansalz von 5-Cyano-(16RS)-16-methyl-18,19-tetradehydro-prostacyclin

Zu einer Lösung von 98 mg 5-Cyano-(16RS)-16-methyl-18,19-tetradehydro-prostacyclin (Herstellung: Beispiel 2) in 3 ml Acetonitril fügt man bei 80°C unter Rühren eine Lösung von 30 mg Tris-(hydroxymethyl)-aminomethan in 0,1 ml Wasser und rührt 16 Stunden bei Raumtemperatur. Man erhält nach Abtrennen des Lösungsmittels 103 mg der Titelverbindung als wachsartige Masse.

**Patentansprüche**

1. 5-Cyano-prostacyclinderivate der allgemeinen Formel I

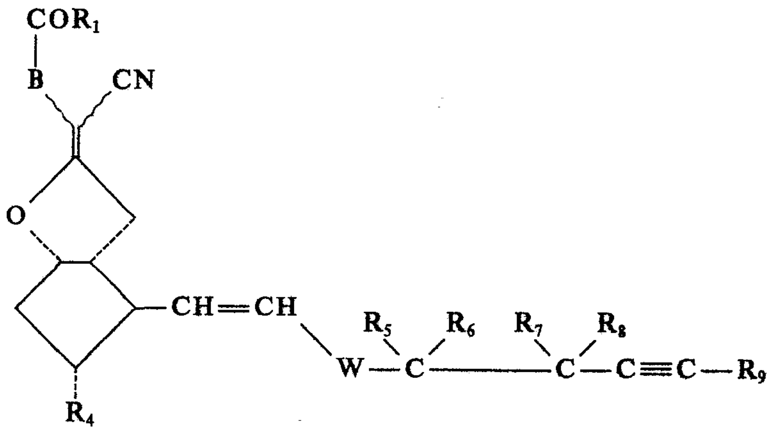

(I)

worin

$R_1$ den Rest $OR_2$, wobei $R_2$ Wasserstoff oder Alkyl mit 1–4 C-Atomen bedeutet, oder den Rest $NHR_3$ mit $R_3$ in der Bedeutung eines Methansulfonyl- oder Alkanoylrestes mit 1–10 C-Atomen oder eines Wasserstoffatoms,
B eine geradkettige oder verzweigtkettige Alkylengruppe mit 1–10 C-Atomen,
W eine freie oder funktionell abgewandelte Hydroxymethylengruppe oder eine freie oder funktionell abgewandelte

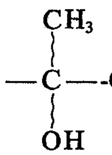 -Gruppe

wobei die OH-Gruppe α- oder β-ständig sein kann,
$R_4$ eine freie oder funktionell abgewandelte Hydroxygruppe,
$R_5$, $R_6$, $R_7$, $R_8$ ein Wasserstoffatom oder eine Alkylgruppe mit 1–5 C-Atomen oder eine Methoxygruppe,
$R_9$ eine Alkylgruppe mit 1–5 C-Atomen und, falls
$R_2$ die Bedeutung eines Wasserstoffatoms hat, deren Salze mit physiologisch verträglichen Basen bedeuten.

2. 5-Cyano-(16RS)-16-methyl-18,19-tetradehydro-prostacyclin.
3. 5-Cyano-(16RS)-16,20-dimethyl-18,19-tetradehydro-prostacyclin.
4. Tris(hydroxymethyl)-aminomethansalz von 5-Cyano-(16RS)-16-methyl-18,19-tetradehydro-prostacyclin.
5. Verfahren zur Herstellung der 5-Cyan-prostacyclinderivate der allgemeinen Formel I, dadurch gekennzeichnet, daß man eine Verbindung der allgemeinen Formel II

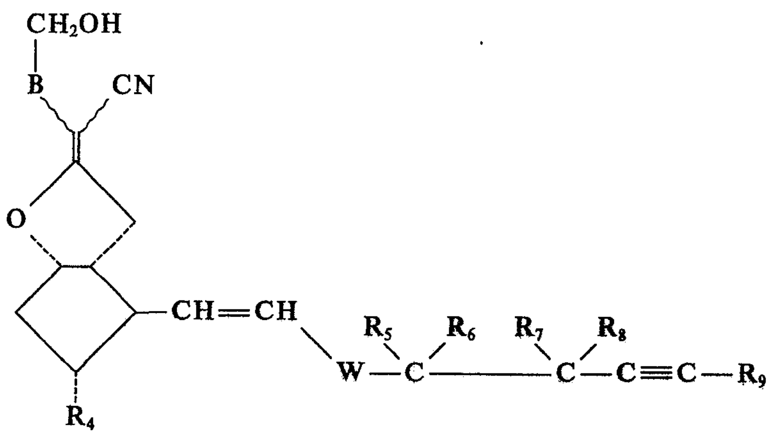

(II)

worin B, $R_5$, $R_6$, $R_7$, $R_8$ und $R_9$ die oben angegebenen Bedeutungen haben und freie Hydroxygruppen in $R_4$ und W geschützt sind, oxidiert und gegebenenfalls anschließend in beliebiger Reihenfolge Isomere trennt, geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe mit Verbindungen der allgemeinen Formel III

$$O=C=N-R_3 \qquad (III)$$

worin $R_3$ die oben angegebene Bedeutung hat, umsetzt oder eine Carboxylgruppe mit einer physiologisch verträglichen Base in ein Salz überführt.

6. Arzneimittel, bestehend aus einer oder mehreren Verbindungen gemäß Anspruch 1 und üblichen Hilfs- und Trägerstoffen.

## Claims

1. 5-cyanoprostacyclin derivatives of the general formula I

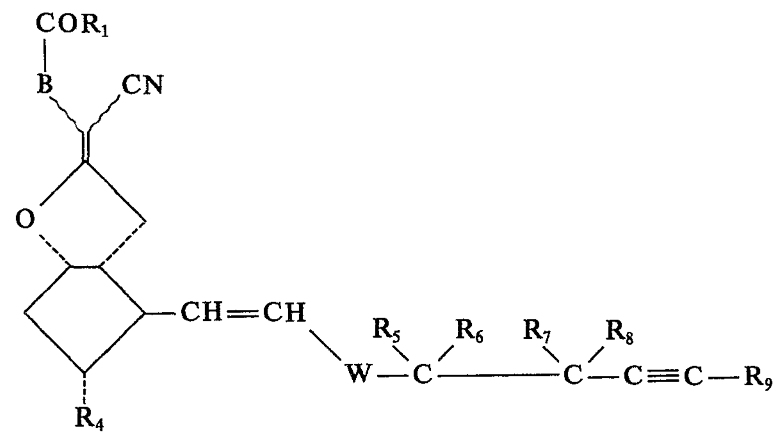

(I)

in which

- $R_1$ represents the radical $OR_2$, in which $R_2$ represents hydrogen or alkyl having from 1 to 4 carbon atoms; or the radical $NHR_3$, in which $R_3$ represents a methanesulphonyl radical or an alkanoyl radical having from 1 to 10 carbon atoms or a hydrogen atom,
- B represents a straight-chain or branched-chain alkylene group having from 1 to 10 carbon atoms,
- W represents a free or funktionally modified hydroxymethylene group or a free or functionally modified

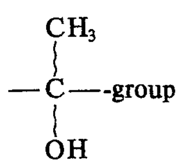


in which the OH group can be in the α-configuration or the β-configuration,
- $R_4$ represents a free or functionally modified hydroxy group,
- $R_5$, $R_6$, $R_7$ and $R_8$ represent a hydrogen atom or an alkyl group having from 1 to 5 carbon atoms or a methoxy group,
- $R_9$ represents an alkyl group having from 1 to 5 carbon atoms and, if
- $R_2$ represents a hydrogen atom, the salts thereof with physiologically tolerable bases.

2. 5-cyano-(16RS)-16-methyl-18,19-tetradehydroprostacyclin.

3. 5-cyano-(16RS)-16,20-dimethyl-18,19-tetradehydroprostacyclin.

4. The tris(hydroxymethyl)-aminomethane salt of 5-cyano-(16RS)-16-methyl-18,19-tetradehydroprostacyclin.

5. Process for the manufacture of the 5-cyanoprostacyclin derivatives of the general formula I, characterised in that a compound of the general formula II

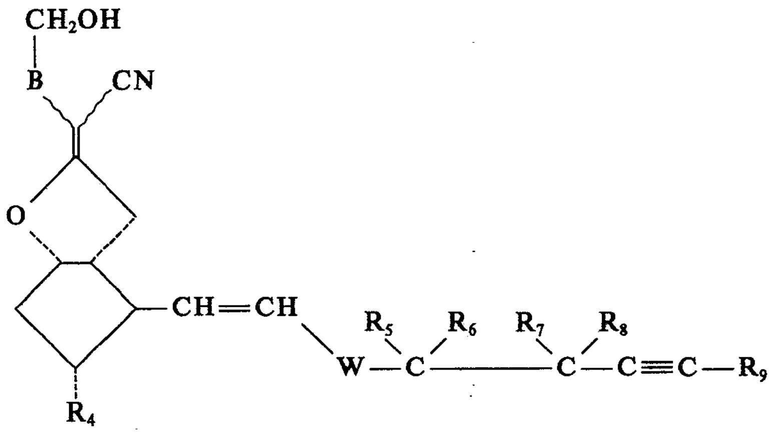

(II)

in which B, $R_5$ $R_6$, $R_7$, $R_8$ and $R_9$ have the above-mentioned meanings and free hydroxy groups in $R_4$ and W are protected, is oxidised and then, optionally, in any desired order, isomers are separated, protected hydroxy groups are freed and/or free hydroxy groups are esterified or etherified and/or a free carboxy group is esterified and/or an esterified carboxy group is hydrolysed or a carboxy group is reacted with compounds of the general formula III

$$O=C=N-R_3 \qquad \text{(III)}$$

in which $R_3$ has the above-mentioned meaning, or a carboxy group is converted into a salt with a physiologically tolerable base.

6. Medicament comprising one or more compounds according to claim 1 and customary auxiliaries and carriers.

**Revendications**

1. Dérivés de la cyano-5 prostacycline qui répondent à la formule générale I

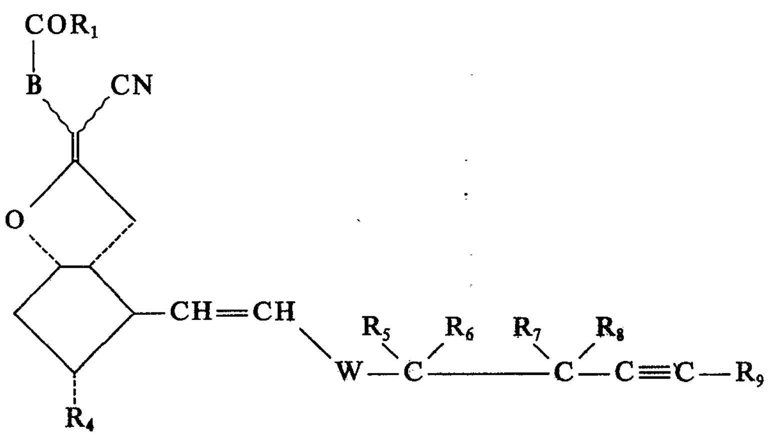

(I)

dans laquelle

$R_1$ représente soit un radical $OR_2$ dans lequel $R_2$ désigne l'hydrogène ou un alkyle en $C_1-C_4$, soit un radical $NHR_3$ dans lequel $R_3$ désigne un radical méthane-sulfonyle, un radical alcanoyle en $C_1-C_{10}$ ou un atome d'hydrogène,

B représente un radical alkylène, linéaire ou ramifié, qui contient de 1 à 10 atomes de carbone,

W représente un radical hydroxyméthylène libre ou sous la forme d'un dérivé fonctionnel ou un radical

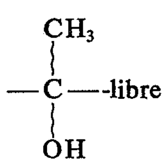

ou sous la forme d'un dérivé fonctionnel, le radical $-OH$ ayant la configuration $\alpha$- ou la configuration $\beta$,

$R_4$ représente un radical hydroxy libre ou sous la forme d'un dérivé fonctionnel,

$R_5$, $R_6$, $R_7$ et $R_8$ représentent chacun un atome d'hydrogène, un alkyle en $C_1-C_5$ ou un méthoxy, et

$R_9$ représente un alkyle en $C_1-C_5$,

et, dans le cas où $R_2$ désigne un atome d'hydrogène, les sels que forment ces composés avec des bases acceptables du point de vue physiologique.

2. Cyano-5 méthyl-16 (16RS) tétradéhydro-18,19 prostacycline.

3. Cyano-5 diméthyl-16,20 (16RS) tétradéhydro-18,19 prostacycline.

4. Sel de tris-(hydroxyméthyl)-aminométhane de la cyano-5 méthyl-16 (16RS) tétradéhydro-18,19 prostacycline.

5. Procédé de préparation des dérivés de la cyano-5 prostacycline répondant à la formule générale I, procédé caractérisé en ce que on oxyde un composé répondant à la formule générale II

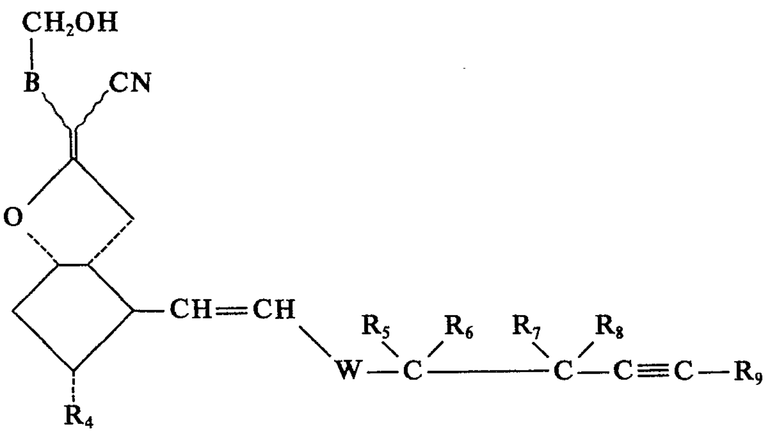

(II)

dans laquelle B, $R_5$, $R_6$, $R_7$, $R_8$ et $R_9$ ont les significations précédemment données et les groupes hydroxy libres dans $R_4$ et W sont protégés, et ensuite, le cas échéant, en opérant dans l'ordre que l'on veut, on sépare les isomères, on libère des groupes hydroxy protégés et/ou on estérifie ou éthérifie des groupes hydroxy libres et/ou on estérifie un radical carboxy libre et/ou on saponifie un radical carboxy estérifié ou on fait réagir un radical carboxy avec des composés répondant à la formule générale (III)

$$O=C=N-R_3 \qquad \text{(III)}$$

dans laquelle $R_3$ a la signification indiquée ci-dessus, ou on transforme un radical carboxy en un sel avec une base acceptable du point de vue physiologique.

6. Médicament constitué d'un ou plusieurs composés selon la revendication 1 et d'adjuvants et excipients usuels.